# EUROPÄISCHE PATENTSCHRIFT

(11) **EP 0 835 325 B1**
(45) Veröffentlichungstag und Bekanntmachung des Hinweises auf die Patenterteilung: **28.02.2001**
(21) Anmeldenummer: 96922735.4
(22) Anmeldetag: 27.06.1996
(51) Int. Cl.: C12Q 1/68, C12Q 1/02, G01N 33/50

(54) **IN VITRO-TESTVERFAHREN ZUM NACHWEIS CHEMIKALIEN-INDUZIERTER EMBRYOTOXISCHER/TERATOGENER EFFEKTE**
IN VITRO TEST PROCEDURE FOR DETECTING CHEMICALLY-INDUCED EMBRYOTOXIC AND TERATOGENIC EFFECTS
METHODE D'ANALYSE IN VITRO POUR METTRE EN EVIDENCE DES EFFETS EMBRYOTOXIQUES/TERATOGENES INDUITS PAR DES SUBSTANCES CHIMIQUES

(30) Priorität: 28.06.1995 DE 19525285
(43) Veröffentlichungstag der Anmeldung: 15.04.1998
(73) Patentinhaber: INSTITUT FÜR PFLANZENGENETIK UND KULTURPFLANZENFORSCHUNG, 06466 Gatersleben (DE)
(72) Erfinder: WOBUS, Anna, Magdalene, D-06466 Gatersleben (DE); FRANZ, Wolfgang-Michael, D-23627 Gro Grönau (DE)
(74) Vertreter: Baumbach, Friedrich
(86) Internationale Anmeldenummer: DE9601183
(87) Internationale Veröffentlichungsnummer: WO9701644

(56) Entgegenhaltungen:
- EP-A- 0 370 813
- WO-A-89/05864
- DD-A- 299 439
- US-A- 5 346 812
- FASEB JOURNAL FOR EXPERIMENTAL BIOLOGY, Bd. 9, Nr. 4, April 1995, BETHESDA, MD US, Seite A942 XP000196437 C-C. LAI: "Genetic engineered mouse embryonic stem (ES) cells for the detection of mutation during the early development"
- TOXICOLOGY IN VITRO, Bd. 8, Nr. 4, 1994, OXFORD GB, Seiten 697-701, XP000196434 D.R. NEWALL ET AL.: "The stem-cell test: a novel in vitro assay for teratogenic potential" in der Anmeldung erwähnt
- GENES AND DEVELOPMENT, Bd. 5, Nr. 9, September 1991, GB, Seiten 1513-1523, XP000562802 G. FRIEDRICH ET AL.: "Promoter traps in embryonic stem cells: a genetic screen to identify and mutate developmental genes in mice"

## Beschreibung

Die Erfindung betrifft ein in vitro-Testverfahren zum Nachweis Chemikalien-induzierter embryotoxischer(z. B. auch teratogener) Effekte auf der Basis differenzierender pluripotenter embryonaler Stamm(ES)-Zellen der Maus und Ratte sowie an aus primordialen Keimzellen etablierten embryonalen Keim(EG)-Zellen.

Der Nachweis teratogener Eigenschaften von chemischen Wirkstoffen erfolgt derzeit durch Bestimmungen der Reproduktionstoxizität von Prüfsubstanzen nach Einfach- und Mehrfachapplikation an trächtigen Laborsäugern und durch Prüfungen der Embryotoxizität in frühen Stadien der Schwangerschaft (Holz und Siegemund, Der Einsatz von Tieren in der Forschung und Entwicklung im Verbraucher- und Umweltschutz, Abschlußbericht zur Basiserhebung des Batelle-Instituts 1988). Des Weiteren werden in vitro-Versuche mit Säuger-Embryonen (Neubert und Merker, Cell culture techniques - applicability for studies on prenatal differentiation and toxicity, de Gruyter, Berlin - New York (1981)) und embryonalen Organen für Teratogenitätsteste eingesetzt. Diese Testverfahren haben jedoch den großen Nachteil, daß sie nach wie vor den Einsatz einer großen Anzahl von lebenden Säugern, insbesondere Ratten und Mäusen voraussetzen. In vitro-Testverfahren, bei denen Primärzellkulturen aus Extremitätenknospen (z.B. "limb buds", Kochhar, Teratology 11, 273-287 (1975)), oder Gehirnanlagen embryonaler Ratten (Flint and Orton, Toxicol. Appl. Pharmacol. 76, 383-395 (1984)) oder permanente Zellinien aus embryonalen oder adulten Säugetiergeweben oder aus menschlichen Geweben, wie Tumorzellen des Ovars oder embryonale Gaumenzellen eingesetzt wurden, erfüllen im engeren Sinn nicht die Anforderungen, die an Teratogenitätstests gestellt werden, nämlich Hinweise auf mögliche Entwicklungsstörungen während der Embryogenese zu geben.

Seit einigen Jahren gibt es Bemühungen, permanente Kulturen totipotenter/pluripotenter embryonaler Stammzellen (ES-Zellen) auch zum Nachweis embryotoxischer und mutagener Stoffe zu verwenden (Laschinski et al, Reproductive Toxicol. 5, 57-65 (1991), Newall and Beedles, Toxicol. in Vitro 8, 697-701 (1994), Sehlmeyer and Wobus, Mutation Res. 324, 69-76 (1994)). Embryonale Stammzellen sind d i e totipotenten Zellen des frühen Embryos, aus denen sich der komplette Säugetierorganismus entwickelt. Störungen während der Keimblatt- und pränatalen Entwicklung können zum Absterben der Embryonen (präimplantativem Tod), zu Entwicklungsstörungen, Fehlentwicklungen bzw. Mißbildungen führen.
Als in vitro-Systeme totipotenter bzw. pluripotenter Zellen sind
1. Embryonale Stammzellen (ES-Zellen), permanente Linien totipotenter embryonaler Zellen (Evans and Kaufman, Nature 282, 507-509 (1981)) und
2. Embryonale Keimzellen ("embryonic germ cells", EG-Zellen), die aus primordialen Keimzellen (PGC) ca. 9 Tage alter Embryonen gewonnen und als permanente Zellinien kultiviert werden (Stewart et al, Dev. Biol. 161,626-628 (1994))
bekannt.

Sowohl ES-Zellen als auch EG-Zellen sind totipotent und nehmen in vivo nach Retransfer in die Blastozyste an der normalen Embryogenese teil und sind in der Lage, die Keimbahn zu kolonisieren (Bradley et al, Nature Bd. 309, 255-256 (1984), Matsui et al, Cell 70, 841-847 (1992)).
In vitro können ES-Zellen nach Differenzierung in Embryo-ähnlichen Aggregaten, sogenannten "embryoid bodies" Derivate aller drei Keimblätter bilden, d.h. in kardiogene (Doetschmann et al, J. Embryol. Exp. Morphol. 87, 27-45 (1985)), myogene (Rohwedel et al, Dev. Biol. 164, 87-101 (1994), neuronale und hämatopoietische Zellen (Wiles and Keller, Development 111, 259-267 (1991)) differenzieren.

In bisherigen Experimenten wurde nachgewiesen, daß das Teratogen Retinsäure (RA) zu gravierenden Veränderungen der Expression gewebespezifischer Gene führt, wenn es zu bestimmten Zeiten der "embryoid body"-Differenzierung einwirkte und zu Aktivierung, Hemmung oder Modulation der Expression von Herz- oder Skelettmuskel-spezifischen Genen führte (Wobus et al., Roux's Arch. Dev. Biol. 204, 1994, 36-45). Diese Aktivierung, Reprimierung oder Modulation der Genexpression kann auch über Reportergene, z. B. X-Gal oder Luziferase, sichtbar gemacht werden. Bisherige in vitro-Verfahren mit ES-Zellen betreffen in erster Linie Testverfahren zum Nachweis der zytotoxischen Wirksamkeit embryotoxischer Verbindungen mit Hilfe des MTT-Testes (Laschinski et al s.o.). Dabei wurde in ES-Zellen eine höhere zytotoxische Sensitivität beobachtet als in differenzierten Zellen.
Untersuchungen zur Zytotoxizität mit Hilfe des "stem cell tests" zum Nachweis teratogener Effektivität wurden von Newall and Beedles, Toxicol. in Vitro 8, 697-701 (1994) beschrieben.
In allen diesen Untersuchungen dienten zytotoxische Effekte als Maß für embryoschädigende Eigenschaften teratogener/embryotoxischer Substanzen.
Es ist festzustellen, daß ES-Zellen das zur Zeit wichtigste Zellmodell in der Entwicklungsbiologie sind, vor allem zur Konstruktion transgener Organismen, ihr Einsatz in der Reproduktions- und Gentoxikologie bisher jedoch begrenzt ist.

Die Erfindung hat das Ziel, ein routinemäßig einsetzbares in vitro-Testverfahren zur Ermittlung Chemikalien-induzierter embryotoxischer/teratogener Effekte bereit zu stellen.

Die Aufgabe der Erfindung liegt darin, ein in vitro-Testverfahren zum Nachweis embryotoxischer/teratogener Eigenschaften von chemischen Wirkstoffen an embryonalen Stamm(ES)-Zellen oder an aus primordialen Keimzellen etablierten embryonalen Keim(EG)-Zellen zu entwickeln. Das Testverfahren soll insbesondere geeignet sein, Hinweise auf mögliche Entwicklungs- und Differenzierungsstörungen während der Embryogenese zu geben.

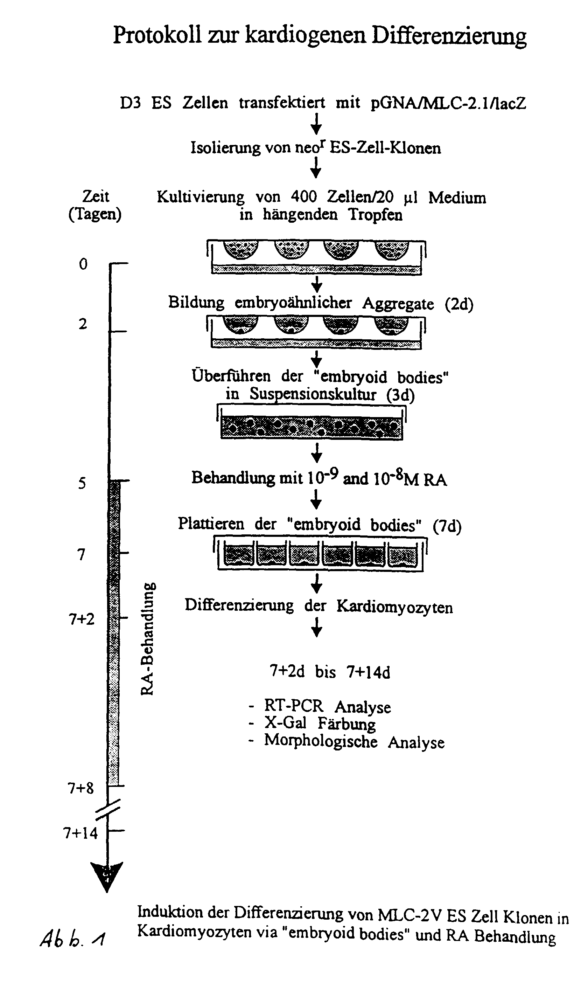

Die Erfindung wird gemäß der Ansprüche realisiert.
Es werden stabile transgene ES- oder EG-Stammzellklone konstruiert, bei denen ein bakterielles Reportergen LacZ oder das Luziferasegen unter die Kontrolle von gewebespezifischen Promotoren oder Entwicklungskontrollgenen gebracht wird. Nach Differenzierung der ES-Zellen in Anwesenheit teratogener Substanzen in die verschiedenen Keimbahnderivate erfolgt eine differenzierungsabhängige Expression in den Zellen, die die gewebespezifischen Promotoren exprimieren. Die Aktivierung, Reprimierung oder Modulation dieser gewebespezifischen Gene wird mit einer einfachen Färbereaktion, dem X-Gal Assay, bestimmt. Mit der Erfindung wird eine Batterie von relevanten Test-ES-Zellklonen entwickelt, die neben dem Reportergen LacZ (und einer Neomycinkassette zur Selektion stabiler Transfektanten) Promotorsequenzen enthalten, die charakteristische und wesentliche Merkmale der ektodermalen und mesodermalen Linie regulieren. Das sollten insbesondere Gene sein, die die neuronale, kardiogene, die Muskel- und die Skelettentwicklung determinieren. Nach Transfektion dieser Vektoren in pluripotente ES-/EG-Zellen der Maus oder der Ratte werden stabile Stammzellklone selektiert und diese nach "embryoid body"-Entwicklung zu spezifischen Zeiten in Anwesenheit von Testsubstanzen differenziert (Abb.1). Danach kann die differenzierungsabhängige Expression der gewebespezifischen Gene mit Hilfe der X-Gal-Färbung in verschiedenen Entwicklungsstadien (unter Zeiteinhaltung und Einsatz dafür geeigneter Vektoren) nachgewiesen werden. Diese Färbetechnik ist standardisierbar und über photometrische Verfahren automatisierbar.
Mit diesem Testverfahren ist es möglich, in vitro Chemikalieninduzierte Veränderungen der zellulären Differenzierung, die während der Keimblattdifferenzierung und frühen embryonalen Entwicklung stattfinden und zu Entwicklungsstörungen führen, festzustellen. Bei dem erfindungsgemäßen Testverfahren werden keine lebenden Tiere, sondern permanente Zellinien eingesetzt.

Der wesentliche Vorteil der Erfindung besteht darin, daß ein standardisierbares, im Routine-Screening einsetzbares in vitro Modell etabliert wird, das zu einer Einsparung von Versuchstieren führt. So wurden im Bereich der Reproduktionstoxikologie in Deutschland pro Jahr (1987) ca. 20 000 Säugetiere und Vögel eingesetzt. Darüber hinaus werden eine große Anzahl von Säugern zur Entnahme embryonaler Organe und Gewebe (embryonale Herzmuskelzellen, "limb bud" Kulturen, "micromass"-Assay u.a.) benötigt, so daß die Gesamtzahl bei Tierzahlen von mindestens 50 000 liegen dürfte.

Mit dem erfindungsgemäßen Verfahren können im Vorscreening folgende Testverfahren teilweise ersetzt und ergänzt werden:
- in vivo-Segment II-Studien,
- Teste mit embryonalen Organen ("limb bud" Kulturen) und Primärkulturen
- Testverfahren mit artifiziellen Embryonen

Anschließend wird die Erfindung an einem Ausführungsbeispiel näher erläutert, das die Anmeldung jedoch nicht beschränken soll.

### Beispiel 1

Das Testverfahren ist in Abb. 1 schematisch dargestellt. Es werden transgene ES-Zellklone, bei denen LacZ unter der Kontrolle des ventrikelspezifischen MCL-2V Promotors (Franz et al, Circ. Res. 73 (1993) 629-638) exprimiert wird, für die Untersuchung solcher kardiotoxischen Substanzen eingesetzt, die zu Entwicklungsstörungen am Herzen führen.
Zum Einsatz kommen Klone, die mit dem Vektor pGNA-MLC 2.1-LacZ transfiziert wurden und MLC-2V stabil exprimieren.
Während der mesodermalen Differenzierung in embryoid bodies wird die Testsubstanz Retinsäure (RA) zugegeben (z. B. von Tag 5 bis 15) und anschließend eine X-Gal-Färbung der differenzierten embryoid bodies durchgeführt.

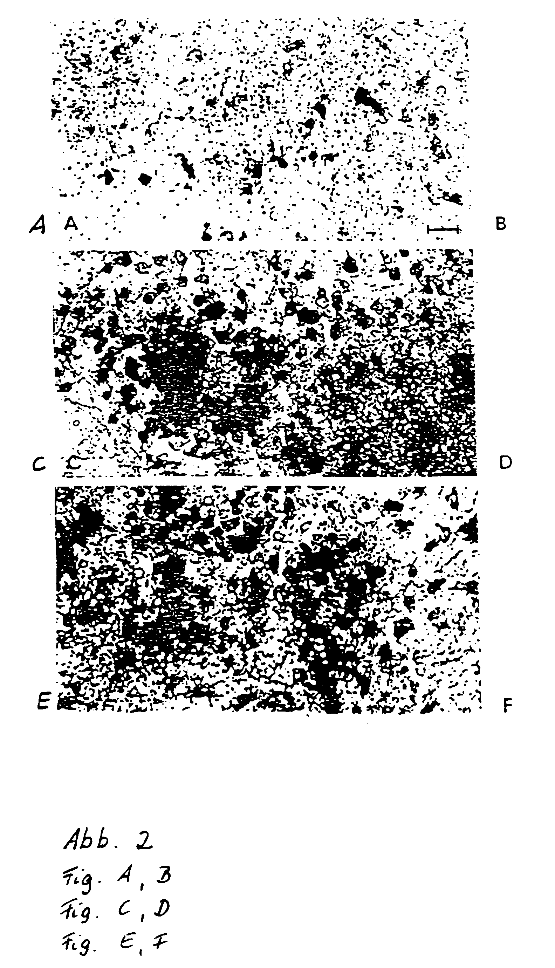

Abb.2 zeigt die Aktivierung der MLC-2V-Expression im MLC-Klon 3 durch 10⁻⁸M (E,F) und 10⁻⁹M (C,D)RA nach Behandlung zwischen 5. und 15. Tag der embryoid body-Differenzierung. Die Kontrollzellen (A,B) zeigen X-Gal-Färbung ohne Induktion durch die teratogene Substanz RA.

Die Vektoren werden auch zur Transfektion von EG-Zellen oder von Ratten-ES-Zellen benutzt.

## Patentansprüche

1. In vitro-Testverfahren zum Nachweis Chemikalien-induzierter embryotoxischer/teratogener Effekte auf der Basis differenzierender pluripotenter embryonaler Stamm(ES)-Zellen oder an aus primordialen Keimzellen etablierten embryonalen Keim(EG)-Zellen der Maus und Ratte durch
- Selektion stabiler transgener ES-/EG-Zell-Klone, welche beliebige Reportergene enthalten, die unter Kontrolle von gewebespezifischen Promotern, die zellspezifische Strukturgene, Transkriptionsfaktoren oder Entwicklungskontrollgene kontrollieren, stehen
- differenzierungsabhängige Expression gewebespezifischer Gene von ES-/EG-Zell-Klonen in Anwesenheit von zu bestimmten Zeiten der in vitro Differenzierung einwirkenden teratogenen Substanzen und nachfolgende Differenzierung in die verschiedenen Keimbahnderivate und
- Nachweis der Chemikalien-induzierten Aktivierung, Reprimierung oder Modulation von gewebespezifischen und die Embryonalentwicklung beeinflussenden Genen anhand der Koexpression der Reportergene.

2. In vitro-Testverfahren nach Anspruch 1, dadurch gekennzeichnet, daß als Reportergene LacZ oder Luciferase verwendet werden.

3. In vitro-Testverfahren nach Anspruch 1 und 2, dadurch gekennzeichnet, daß die Zell-Klone
- das Reportergen LacZ und eine Neomycinkassette zur Selektion stabiler Transfektanten sowie
- Promotorsequenzen von Genen, die charakteristische und wesentliche Merkmale der ektodermalen und mesodermalen Linie kodieren,
enthalten.

4. In vitro-Testverfahren nach Anspruch 1 und 2, dadurch gekennzeichnet, daß als Promotoren solche eingesetzt werden, die gewebespezifische Gene kodieren, wie z.B. Gene, die die neuronale Herz-, Muskel- oder Skelettentwicklung determinieren, oder Promotoren anderer Entwicklungskontrollgene (Hox oder Pax-Gene).

5. In vitro-Testverfahren nach Anspruch 1 und 2, dadurch gekennzeichnet, daß im Verlauf der Differenzierung durch exogene Testsubstanzen die Reportergenkonstrukte spezifisch aktiviert, reprimiert oder moduliert werden und daß die differenzierungsabhängige Expression im Testverfahren über "embryoid body" - Differenzierung in verschiedene Linien, siehe 4, erfolgt.

6. In vitro-Testverfahren nach Anspruch 1 - 6, dadurch gekennzeichnet, daß durch Einwirkung von Chemikalien das Expressionsmuster der Promotor-Reportergenkonstrukte beeinflußt und mit Hilfe der X-Gal-Färbung bestimmt wird.

## Claims

1. In-vitro testing method for proving chemical-induced embryotoxic/teratogenic effects on the basis of differentiating pluripotent embryonal parent (ES) cells, or embryonal gametes (EG) established from primordial gametes in mice and rats through
- selection of stable transgenic ES/EG cell clones containing any reporter genes which are under the control of tissue-specific promoters, in turn controlling cell-specific structural genes, transcription factors or developmental control genes,
- differentiation-dependent expression of tissue-specific genes of ES/EG cell clones in the presence of teratogenic substances acting at specific times of the in-vitro differentiation and subsequent differentiation in the various embryo path derivatives, and
- proof of chemical-induced activation, repression or modulation of tissue-specific genes and genes influencing the embryonal development with reference to the coexpression of the reporter genes.

2. In-vitro testing method as claimed in Claim 1, characterised in that LacZ or luciferase are used as reporter genes.

3. In-vitro testing method as claimed in Claims 1 and 2, characterised in that the cell clones contain
- the reporter gene LacZ and a neomycin cassette for selection of stable transfectants, and
- promoter sequences of genes which code the characteristic and essential features of the ectodermal and mesodermal line.

4. In-vitro testing method as claimed in Claims 1 and 2, characterised in that the promoters used are those which code tissue-specific genes such as genes that determine neuronal heart, muscle or skeletal development, or promoters of other developmental control genes (hox or pax genes).

5. In-vitro testing method as claimed in Claims 1 and 2, characterised in that in the course of differentiation by exogenic test substances the reporter gene constructs are specifically activated, repressed or modulated, and in that the differentiation-dependent expression occurs in the testing method by means of 'embryonic body' differentiation in different lines, as in 4.

6. In vitro testing method as claimed in Claims 1 to 6, characterised in that through development of chemicals the expression pattern of the promoter/reporter gene constructs is influenced and determined with the assistance of X Gal colouring.

## Revendications

1. Méthode de test *in vitro* pour la mise en évidence d'effets embryotoxiques ou tératogènes induits par des produits chimiques, basé sur des cellules souches embryonnaires (CSE) multipotentes différenciées ou sur des cellules germinales embryonnaires (CGE) établies à partir de cellules germinales primordiales de rat et de souris, comportant les étapes de :
- sélection de clones transgéniques stables de cellules CSE/CGE contenant des gènes *reporters* quelconques, qui sont contrôlés par des promoteurs spécifiques des tissus contrôlant des gènes de structure, des facteurs de transcription ou des gènes de contrôle du développement spécifiques des cellules,
- expression, en fonction de la différenciation, de gènes spécifiques des tissus de clones de cellules CSE/CGE en présence de substances tératogènes agissant à des stades donnés de la différenciation *n vitro* et différenciation ultérieure dans les différents dérivés du génotype et
- mise en évidence de l'activation, de la répression ou de la modulation induite par les produits chimiques au niveau de gènes spécifiques des tissus et influençant le développement embryonnaire, par co-expression des gènes *reporters.*

2. Méthode de test *in vitro* selon la revendication 1, caractérisé en ce qu'on utilise comme gènes *reporters* LacZ ou la luciférase.

3. Méthode de test *in vitro* selon les revendications 1 et 2, caractérisée en ce que les clones cellulaires contiennent :
- le gène *reporter* LacZ et une cassette de néomycine en vue de la sélection des transfectants stables, et
- des séquences de promoteur de gènes codant des traits caractéristiques et essentiels des lignées ectodermique et mésodermique.

4. Méthode de test *in vitro* selon les revendications 1 et 2, caractérisée en ce que les promoteurs utilisés sont aptes à coder des gènes spécifiques des tissus, par exemple des gènes déterminant le développement neuronal du coeur, des muscles ou du squelette ou les promoteurs d'autres gènes contrôlant le développement (gènes Hox ou Pax).

5. Méthode de test *in vitro* selon les revendications 1 et 2, caractérisée en ce qu'au cours de la différenciation, les constructions de gènes *reporters* sont spécifiquement activées, inhibées ou modulées par les substances exogènes testées et en ce que l'expression dépendante de la différenciation se fait par différenciation en « corps embryoïde » (voir 4) dans la méthode d'essai.

6. Méthode d'essai selon les revendications 1 à 6, caractérisée en ce que l'action de produits chimiques influence le schéma d'expression des constructions de gènes *reporters* et de promoteurs et peut être déterminée à l'aide de la coloration X-Gal.
